# EUROPEAN PATENT SPECIFICATION

(11) **EP 0 340 304 B1**
(45) Date of publication and mention of the grant of the patent: **13.07.1994**
(21) Application number: 88900583.1
(22) Date of filing: 25.12.1987
(51) Int. Cl.: A61M 25/00

(54) **GUIDE WIRE FOR CATHETERS AND METHOD OF MANUFACTURING SAME**
FÜHRUNGSDRAHT FÜR KATHETER UND VERFAHREN ZUR HERSTELLUNG
FIL DE GUIDAGE POUR CATHETERS ET PROCEDE DE PRODUCTION

(30) Priority: 07.01.1987 JP 1468/87
(43) Date of publication of application: 08.11.1989
(73) Proprietor: TERUMO KABUSHIKI KAISHA, Tokyo 151 (JP)
(72) Inventor: SAGAE, Kyuta, Fuji-shi Shizuoka-ken 417 (JP); SUGIYAMA, Yoshiaki, Fuji-shi Shizuoka-ken 417 (JP)
(74) Representative: Henkel, Feiler, Hänzel & Partner
(86) International application number: JP8701031
(87) International publication number: WO8804940

(56) References cited:
- EP-A- 0 141 006
- WO-A-85/01444
- JP-A- 5 948 643
- JP-A- 6 063 066
- US-A- 3 789 841

## Description

### Technical Field

The present invention relates to a method of making a catheter guide wire for guiding a clinical or testing catheter to a predetermined portion of a body cavity such as a blood vessel, a digestive tract, and a windpipe and holding it therein.

### Prior Art

When a catheter is to be guided to a branching peripheral portion of a blood vessel or the like, first, a guide wire must be guided to a target portion. In this case, since a target portion is generally thin and thus tends to be easily damaged, the distal end portion of the guide wire must be flexible so that it will not damage a blood vessel wall, will follow the shape of the blood vessel well even if the blood vessel is curved, and can be inserted in a complex branching blood vessel. Meanwhile, the proximal end portion of the guide wire must have torque transmitting performance so that a manual operation performed at the proximal end portion is transmitted to the distal end portion. Thus, the proximal end portion of the guide wire must have comparatively high rigidity.

According to a conventional catheter guide wire having the above characteristics, a coil guide wire is made of a stainless steel wire or a piano wire, or a guide wire is made of a plastic monofilament. In each of these guide wires, its sectional area is decreased from its proximal to distal end portion, and the guide wire forms a main portion having relatively high rigidity and a relatively flexible distal end portion.

However, plastic deformation can easily occur in these conventional guide wires, and some manual operation can kink the guide wires. A kinked portion becomes an obstacle during introduction of a catheter, thus rendering smooth introduction operation of a catheter impossible as well as greatly degrading its torque transmitting performance.

A catheter guide wire free from such kinking deformation uses a very elastic alloy (e.g., Ni-Ti alloy) as a core member (see Japanese Patent Disclosure (Kokai) No. 60-63066).

A guide wire using a very elastic alloy is flexible and can restore its original shape after it is deformed to a considerable degree (strain of about 8%). Therefore, such a guide wire cannot be easily broken during operation and will not easily attain a bending tendency. However, such guide wire has a high elasticity at its distal end portion and is thus infavorable in terms of flexibility. Then the diameter of its proximal end portion is 0.5 mm or less, the rigidity is insufficient and the torque transmitting performance is poor.

EP-A-0 141 006 describes a guide wire for a catheter having a body portion comparatively high in rigidity and a distal end portion comparatively flexible. At least portions of the body portion and the distal end portion are formed of a super-elastic metallic member. At least portions of inner core portions on the body portion's side and on the distal end portion's side, both of which are coated by a plastic, are formed of the super-elastic metallic member, and at least a portion of the inner core portion on the distal end portion's side is made smaller in cross-section than the inner core portion on the body portion's side. The guide wire has a yield stress in the range of 10 to 80 kg/mm² which could be varied by heat-treatment.

WO-A- 85/01444 describes a guiding mandrel for venous or arterial catheters, gastric probes, puncture cannulas or similar instruments, comprised of a wire core enveloped in a sheathing. The sheathing is comprised, in the case of a sheathing based on helically wound wire, of a plurality of single wires arranged next to each other, helically surrounding the wire core and flat inclined with respect to the axis of the mandrel, respectively the wire core is reduced, in the case of a sheathing based on synthetic material, approximately up to the proximal end of the sheathing.

### Disclosure of the Invention

The present invention has been made in view of the above situation and has as its object to provide a method of making a catheter guide wire wherein its distal end portion is very flexible, buckling deformation is difficult to occur, and its proximal end portion is very rigid, thus having a good torque transmitting performance to the distal end portion.

In order to solve the above problems, according to the present invention, a wire member made of an elastic alloy, and preferably a very elastic alloy, is used as a core member of a catheter guide wire and subjected to a heat treatment by changing the treatment conditions along its longitudinal direction, so that the rigidity at its proximal end portion becomes comparatively high, the flexibility at its distal end portion is increased, and kinking deformation will not easily occur in its distal end portion.

More specifically, according to the present invention, there is provided a method of making a catheter guide wire as defined in claim 1.

Note that the catheter guide wire can be fabricated by using as a core member a wire member made of an elastic alloy member subjected to the heat treatment described above and forming a cover layer of a thermoplastic resin on the core member.

The core member preferably uses a very elastic alloy such as an Ni-Ti alloy, a Cu-Zn-Aℓ alloy, a Cu-Aℓ-Ni alloy, and an Fe-Mn alloy. The core member is preferably tapered such that a diameter at its distal end portion is smaller than that at its proximal end portion. A contrast medium such as a tungsten powder can be added to the thermoplastic resin layer.

A flexible coil spring having an outer diameter equal to or smaller than a minimum inner diameter of the catheter can be mounted to surround at least the distal end portion of the wire member.

In this case, the coil spring is preferably made of a material having a high X-ray impermeability in order to allow an X-ray photographing to be easily confirmed. Therefore, the presence of the coil spring is advantageous in giving a sufficient thickness in an X-ray image without badly affecting the flexibility of the guide wire.

As a result, the coil spring is made of a material selected from a group consisting of stainless steel, platinum, a platinum alloy and a palladium alloy, and preferably has a thickness of 0.01 to 0.15 mm, more preferably 0.05 to 0.1 mm.

In a conventional catheter guide wire, a diameter at a proximal end portion of a wire member made of an elastic alloy or a very elastic alloy is merely increased, and a diameter at its distal end portion is relatively decreased, thereby making the proximal end portion rigid and the distal end portion flexible. Unlike such a conventional catheter guide wire, according to the present invention, a wire member is subjected to a heat treatment by sequentially changing the confunction along its longitudinal direction. As a result, the physical characteristics of the wire member can be set in an ideal state as a catheter guide wire.

### Brief Description of the Drawings

Fig. 1 is a sectional view of a catheter guide wire made according to an embodiment of the present invention;
Fig. 2 is a graph of strain-stress curves of the core member of the guide wire according to the embodiment of the present invention; and
Figs. 3 and 4 respectively represent a sectional view of a catheter guide wire on which a coil spring is mounted made according to another embodiment of the present invention.

### Best Mode for carrying out the Invention

Preferred embodiments of the present invention will be described with reference to the accompanying drawings.

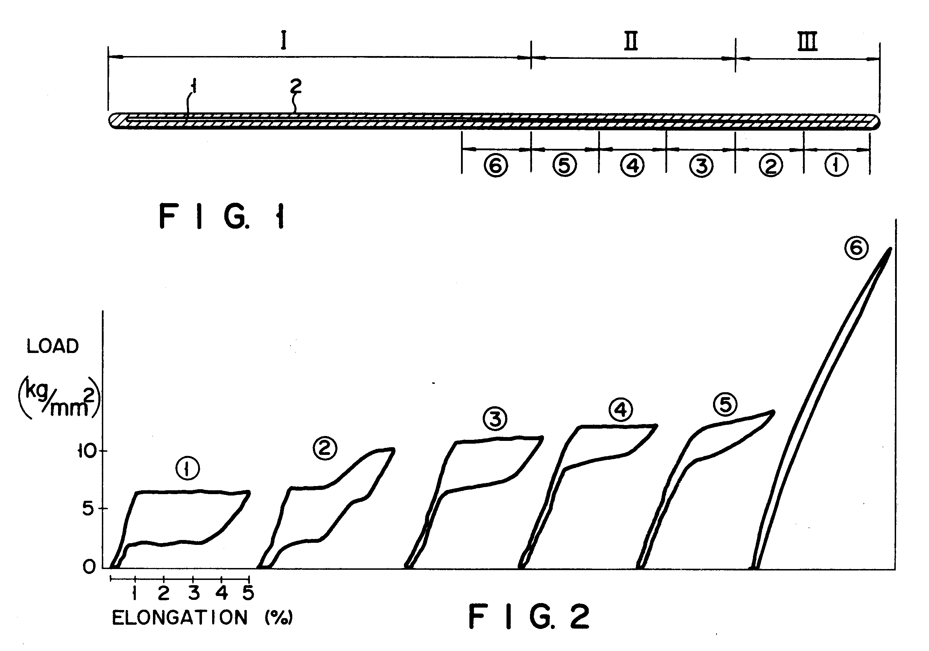

Fig. 1 is a sectional view of a catheter guide wire taken along the longitudinal direction according to an embodiment of the present invention. Referring to Fig. 1, reference numeral 1 denotes a core member; and 2, a thermoplastic resin layer entirely covering core member 1.

Core member 1 is a wire member made of an elastic alloy wire such as a piano wire, and preferably a very elastic alloy such as an Ni-Ti alloy. Core member 1 can have a uniform diameter of 0.2 to 0.4 mm, or can be tapered toward its distal end such that the diameter at its proximal end portion is 0.2 to 0.4 mm and the diameter at its distal end portion is 0.01 to 0.1 mm. In this specification, a very elastic alloy is defined as an alloy whose recoverable elastic strain is as large as several % to more than ten % and whose stress level does not exceed a predetermined value even if the strain is increased. The very elastic alloy generally comprises an Ni-Ti, Cu-Zn-Aℓ, Cu-Aℓ-Ni, or Fe-Mn alloy. If an Ni-Ti alloy is employed, it preferably contains 49 to 58 atm% of Ni and a balance of Ti, and more preferably 49 to 51 atm% of Ni and a balance of Ti. If a Cu-Zn-Aℓ alloy is employed, it preferably contains 38.5 to 41.5 wt% of Zn, 1 to 10 wt% of ADP, and a balance of Cu. If a Cu-Aℓ-Ni alloy is employed, it preferably contains 14 to 14.5 wt% of Aℓ, 3 to 4.5 wt% of Ni, and a balance of Cu. If an Fe-Mn alloy is employed, it preferably contains 28 to 32 wt% of Mn, 6 wt% of Si, and a balance of Fe. A heat treatment is performed by changing the treatment conditions. As a result, the guide wire can have the following physical characteristics in its areas (1) to (III) as shown in Fig. 1.

### (1) Proximal end portion (I)

When the guide wire is guided from, e.g., a straight great blood vessel (e.g., a descending aorta) to an arteriole (e.g., a coronary artery), proximal end a comparatively small number of bent portions. Proximal and portion (I) has a comparatively high rigidity and is difficult to deform. Therefore, forward/backward movement and rotation externally applied to the catheter can be easily transmitted to the distal end portion (II - III) through a blood vessel retaining an introducer (not shown).

### (2) Intermediate portion (II)

Intermediate portion (II) has an elasticity so that it can easily follow a blood vessel curve of a comparatively large curve and can return to its initial shape when deformation caused by the curve is removed. Although it is flexible, intermediate portion (II) hardly attains a bending tendency and is difficult to break.

### (3) Distal end portion (III)

When distal end portion (III) is inserted in a small, curved blood vessel, it can easily follow the blood vessel shape due to it's flexibility, and thus will not damage the blood vessel wall. When a blood vessel has phatologic factor such as arteriosclerosis, the flexibility of distal end portion (III) is important.

Thermoplastic resin layer 2 is provided as needed in order to protect the inner surface of the blood vessel, to prevent formation of thrombus on an outer surface of the guide wire during operation of the guide wire, and not to form a difference in outer diameter between the proxital end portion and the distal end portion. For example, saturated aliphatic polyether urethane is used to form layer 2. A contrast medium can be mixed in the thermoplastic resin in advance in order to increase the contrast of the guide wire through X-ray photographing. For example, 40 to 600 parts by weight (with respect to 100 parts by weight of thermoplastic resin) of a tungsten powder can be mixed as the contrast medium. Note that saturated aliphatic polyether polyurethane is favorable for compounding of tungsten.

Fig. 2 shows the physical characteristics (strain-stress curve) at the respective portions of the core member made according to the present invention after a heat treatment. A heat treatment can be performed in an atmosphere of an inert gas (Ar or He), vacuum (× 10-2 Torr or less) or outer atmosphere. Although a heat treatment can be performed in an outer atmosphere, it is preferably performed in a vacuum in view of embrittlement of the material, and more preferably in an inert gas. The values in Fig. 2 are obtained by cutting the core member sample into 70-mm long pieces starting from its distal end and subjecting the respective samples to a tension test.
Core member: Ni-Ti alloy wire (diameter: 0.4 mm) (49 atm% of Ni and a balance of Ti)

| Heat treatment conditions: | | |
|---|---|---|
| Area of Guide Wire | Heat Treatment Conditions | Tension Test Sample No. |
| Distal end portion (III) | About 2 hrs. at 400 to 500°C and about 24 hrs. at 200°C (in outer atmosphere) | (1)(2) |
| Intermediate portion (II) | About 2 hrs. at 400 to 500°C (in outer atmosphere) | (3)(4)(5) |
| Proximal end portion (I) | No heat treatment after cold rolling | (6) |

The physical characteristics at the respective portions of core member 1 are not limited to those shown in Fig. 2 and can be arbitrarily adjusted and selected in accordance with specific applications.

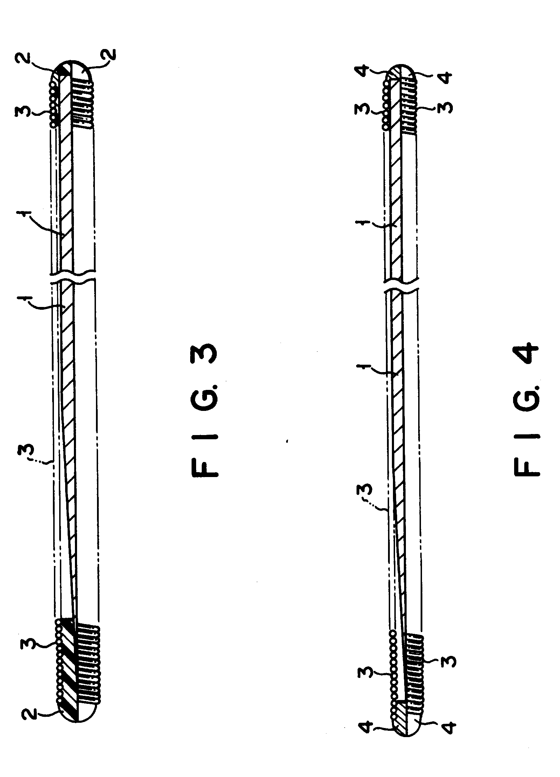

Fig. 3 is a partial sectional view of a catheter guide wire made according to another embodiment of the present invention. Thermoplastic resin layer 2 is formed on the entire surface of core member 1 in the same manner as in Fig. 1, and coil spring 3 having a thickness of 0.08 mm is mounted on an outer surface of resin layer 2 excluding its leading and trailing end faces. Note that coil spring 3 may be provided at only the distal end portion of the guide wire. The outer diameter of the guide wire may be conveniently selected to conform with the inner diameter of a blood vessel to be inserted. Generally, however, the outer diameter of the guide wire may be selected within a range of from 0.2 to 2.0 mm.

When coil spring 3 is applied on resin layer 2 in this manner, the physical characteristics of the guide wire are as flexible at its distal end portion as shown in Fig. 1 and highly resistive to buckling deformation due to the high flexibility of the coil spring 3, relatively high in rigidity at its proximal end portion and excellent in X-ray photographing.

Coil spring 3 can be provided to directly surround core member 1 without intervening thermoplastic resin layer 2.

Fig. 4 shows an example of such a structure of the guide wire, wherein the coil spring 3 is directly wound around the outer wall of core member 1, with its distal and proximal end portions being fixed to core member 1 through a soldering material 4 made for example of Sn-Ag (96:4) alloy.

As described above, according to the catheter guide wire made according to the present invention, a wire member made of an elastic alloy is used as a core member and subjected to a heat treatment by sequentially changing the treatment conditions along its longitudinal direction. As a result, the proximal end portion of the guide wire has predetermined rigidity required in accordance with its application, and its distal end portion has predetermined flexibility.

### Industrial Application

The guide wire made as proposed by this invention is useful for guiding a clinical or testing catheter to a predetermined portion of a body cavity such as blood vessel, a digestive tract and a windpipe, and holding it therein for a period of time.

## Claims

1. A method of making a catheter guide wire for guiding a catheter, wherein the catheter has an inner passage therein, the inner passage having a minimum inner diameter, said method comprising the steps of:
forming by cold rolling a very elastic wire member (1) made of Ti-Ni alloy as a base material, said very elastic wire member (1) having a proximal end portion (I) and a leading end side; and
selectively heat-treating said very elastic wire member so as to make said leading end side comparatively flexible;
which is characterized in that said step of heat-treatment is preceded by the step of dividing said leading end side of said base material along the length thereof into a distal end portion (III) and an intermediate portion (II), at least said distal end portion (III) having an outer diameter equal to or smaller than the minimum inner diameter of the inner passage of said catheter; and that
said step of heat-treatment is performed by heat-treating said distal end portion (III) and said intermediate portion (II) of said base material (1), exept for said proximal end portion (I), at a temperature of 400 to 500°C for about 2 hours, and then subjecting only said distal end portion (II) to a secondary heat-treatment at a temperature of about 200°C for about 24 hours, whereby said proximal end portion (I) is not heat-treated after said cold rolling.

2. A method according to claim 1, characterized in that said wire member (1) is formed so as to have a proximal end portion (I) having an outer diameter ranging from 0.2 to 0.4 mm.

## Patentansprüche

1. Verfahren zur Herstellung eines Katheterführungsdrahts zum Führen eines Katheters mit Innenpassage eines Mindestinnendurchmessers in folgenden Stufen:
Ausbilden durch Kaltwalzen einer hochelastischen Drahtkomponente (1) aus einer Ti-Ni-Legierung als Grundstoffmaterial, wobei die hochelastische Drahtkomponente (1) einen proximalen Endteil (1) und ein vorderes Ende aufweist und
selektives Wärmebehandeln der hochelastischen Drahtkomponente, um das vordere Ende relativ flexibel zu machen;
dadurch gekennzeichnet, daß der Wärmebehandlungsstufe eine Stufe vorgeschaltet wird, in der das vordere Ende des Grundstoffmaterials längs seiner Länge in einen distalen Endteil (III) und einen Zwischenteil (II) unterteilt wird, wobei zumindest der distale Endteil (III) einen Außendurchmesser kleiner oder gleich dem Mindestinnendurchmesser der Innenpassage des Katheters aufweist, und daß
die Wärmebehandlungsstufe derart durchgeführt wird, daß der distale Endteil (III) und der Zwischenteil (II) des Grundstoffmaterials (1) mit Ausnahme seines proximalen Endteils (I) etwa 2 h lang auf eine Temperatur von 400 bis 500°C erwärmt werden und anschließend lediglich der distale Endteil (II) etwa 24 h lang einer zweiten Wärmebehandlung bei einer Temperatur von etwa 200°C unterworfen wird, wobei der proximale Endteil (I) nach dem Kaltwalzen nicht wärmebehandelt wird.

2. Verfahren nach Anspruch 1, dadurch gekennzeichnet, daß die Drahtkomponente (1) derart ausgebildet ist, das sie einen proximalen Endteil (1) eines Außendurchmessers im Bereich von 0,2 bis 0,4 mm aufweist.

## Revendications

1. Procédé de fabrication d'un fil de guidage de cathéter pour guider un cathéter, dans lequel le cathéter comporte un passage intérieur, le passage intérieur ayant un diamètre intérieur minimal, ledit procédé comprenant les étapes consistant :
- à former par laminage à froid un fil très élastique (1) formé d'alliage Ti-Ni en tant que matériau de base, ledit fil très élastique (1) comportant une partie d'extrémité proximale (I) et un côté d'extrémité avant ; et
- à traiter thermiquement de façon sélective ledit fil très élastique de manière à réaliser le côté d'extrémité avant relativement flexible ; **caractérisé** en ce que ladite étape de traitement thermique est précédée par l'étape consistant à diviser ledit côté d'extrémité avant dudit matériau de base le long de sa longueur en une partie d'extrémité distale (III) et une partie intermédiaire (II), au moins ladite partie d'extrémité distale (III) ayant un diamètre extérieur égal ou inférieur au diamètre intérieur minimum du passage intérieur dudit cathéter ; et en ce que
on effectue ladite étape de traitement thermique en traitant thermiquement ladite partie d'extrémité distale (III) et ladite partie intermédiaire (II) dudit matériau de base (1), sauf en ce qui concerne la partie d'extrémité proximale (I), à une température de 400 à 500°C pendant environ 2 heures, et en soumettant ensuite seulement la partie d'extrémité distale (II) à un traitement thermique secondaire à une température d'environ 200°C pendant environ 24 heures, grâce à quoi ladite partie d'extrémité proximale (I) n'est pas traitée thermiquement après ledit laminage à froid.

2. Procédé selon la revendication 1, **caractérisé** en ce que ledit fil (1) est formé de manière que sa partie d'extrémité proximale (I) ait un diamètre extérieur compris entre 0,2 et 0,4 mm.
